# EUROPÄISCHE PATENTANMELDUNG

(11) **EP 1 362 583 A1**
(43) Veröffentlichungstag der Anmeldung: **19.11.2003**
(21) Anmeldenummer: 02010190.3
(22) Anmeldetag: 15.05.2002
(51) Int. Cl.: A61K 9/50, A61K 31/495, A61P 33/10

(54) **Verfahren zur Geschmacksmaskierung von Substanzen durch Mikroverkapselung**

(71) Anmelder: CUM Taste Masking AG, 6370 Stans (CH)
(72) Erfinder: Mattern, Claudia, Dr., 6370 Stans (CH); Berger, Andreas, Dr., 5600 Lenzburg (CH)
(74) Vertreter: Hiebsch, Gerhard F., Dipl.-Ing.

(57) **Zusammenfassung**

Verfahren zur Geschmacksmaskierung von Substanzen durch Mikroverkapselung mit den folgenden Schritten:
a) Herstellung einer Lösung, Emulsion oder Suspension der Wirkstoff(e) in einem geeigneten Medium;
b) Herstellung einer Lösung, Emulsion oder Suspension, die wenigstens ein kapselwandbildendes Material enthält, in einem geeigneten Medium;
c) Zuführen der Lösung, Emulsion oder Suspension aus Schritt (a) zur inneren Düse einer Doppeldüse;
d) Zuführen der Lösung, Emulsion oder Suspension aus Schritt (b) zur äußeren Düse der Doppeldüse;
e) gleichzeitiges Ausstoßen der Lösung, Emulsion oder Suspension aus Schritt (a) und derjenigen aus Schritt (b) aus der Doppeldüse unter Bedingungen, die eine wirksame Tropfenbildung ermöglichen; und
f) Unterwerfen der in Schritt (e) gebildeten Tropfen unter Bedingungen zum Bewirken der Kapselwandbildung:

## Beschreibung

Die Erfindung betrifft ein Verfahren zur Geschmacksmaskierung von Substanzen durch Mikroverkapselung, mit diesem Verfahren herstellbare Mikrokapseln, solche Mikrokapseln enthaltende pharmazeutische Zusammensetzungen sowie die Verwendung von spezielle pharmazeutische Wirkstoffe enthaltenden Mikrokapseln zur Herstellung von Arzneimitteln.

Die Geschmacksmaskierung von Substanzen ist auf unterschiedlichen Gebieten seit Jahrzehnten ein wichtiges Thema. Insbesondere in der Pharmazie stellt sich häufig das Problem, dass pharmazeutische Wirkstoffe nur dann oral verabreichbar sind, wenn ihr unangenehmer Geschmack ausreichend maskiert ist, um vom menschlichen oder tierischen Patienten toleriert zu werden. Eine effektive Geschmacksmaskierung ist insbesondere dann erforderlich, wenn es zur oralen Verabreichungsform keine gleichwertige Alternative gibt, da andere Verabreichungsformen nicht zur Verfügung stehen, nicht so effektiv sind oder für den Patienten unangenehm oder unbequem sind.

Eine ausreichende Geschmacksmaskierung ist dann von besonderer Bedeutung, wenn die Patienten besonders geschmacksempfindlich sind, beispielsweise (im Humanbereich) Kinder oder alte Leute oder aber (im Veterinärbereich) bestimmte Tierarten, wie Hunde, ganz besonders aber Katzen, die etwa 400-mal geschmacksempfindlicher sind als Menschen. Die Effektivität einer Geschmacksmaskierung ist natürlich umso bedeutsamer je unangenehmer der Geschmack und/oder je geschmacksempfindlicher der Patient ist.

Besonders extreme Beispiele sind die anthelmintischen Wirkstoffe Praziquantel und Epsiprantel, die sich durch einen besonders unangenehmen bitteren Geschmack auszeichnen. Praziquantel und Epsiprantel sind andererseits sehr wirksame Wurmmittel im Veterinärbereich und werden insbesondere für regelmäßige Wurmkuren von Hunden und Katzen eingesetzt.

Aus dem Stand der Technik ist eine Vielzahl von Vorschlägen zur Geschmacksmaskierung bekannt. Diese reichen von der Überdeckung des unangenehmen Geschmacks durch Aromastoffe und/oder Zucker über das Einbinden des Wirkstoffes in Polymermatrizes, wie bspw. auf der Basis von Methacrylatpolymeren, das Abfüllen in Kapseln oder das Umhüllen mit Überzügen bis hin zur Mikroverkapselung.

Bei der Mikroverkapselung unterscheidet man im wesentlichen vier Verfahren, nämlich Phasentrennverfahren, mechanisch-physikalische Verfahren, Grenzflächen-Polymerisationsverfahren und molekulare Verkapselung.

Mit keinem dieser Mikroverkapselungsverfahren bzw. keinem anderen bekannten Verfahren zur Geschmackmaskierung ist es aber bisher gelungen, vom Geschmack her besonders kritische Wirkstoffe, wie bspw. Praziquantel oder Epsiprantel, so wirkungsvoll zu maskieren, dass eine problemlose orale Verabreichung möglich ist, da bei diesen bereits geringste Spuren von im Mund- oder Rachenbereich freigesetztem Wirkstoff ausreichend sind, um eine Akzeptanz beim Patienten zu erschweren oder zu verhindern.

Darüber hinaus führen viele der im Stand der Technik bekannten Verfahren zur Geschmacksmaskierung zu Darreichungsformen der Wirkstoffe, die die Bioverfügbarkeit derselben beeinträchtigen.

Der vorliegenden Erfindung liegt daher die Aufgabe zugrunde, ein Verfahren zur Geschmacksmaskierung durch Mikroverkapselung mit einer zumindest nahezu 100%igen Effektivität zur Verfügung zu stellen, wobei darüber hinaus bevorzugt auch noch eine bessere Bioverfügbarkeit der eingekapselten pharmazeutischen Wirkstoffe erreicht werden soll.

Erfindungsgemäß wird diese Aufgabe durch ein Verfahren zur Geschmacksmaskierung von Substanzen durch Mikroverkapselung mit den folgenden Schritten gelöst:
a) Herstellung einer Lösung, Emulsion oder Suspension der Wirkstoff(e) in einem geeigneten Medium;
b) Herstellung einer Lösung, Emulsion oder Suspension, die wenigstens ein kapselwandbildendes Material enthält, in einem geeigneten Medium;
c) Zuführen der Lösung, Emulsion oder Suspension aus Schritt (a) zur inneren Düse einer Doppeldüse;
d) Zuführen der Lösung, Emulsion oder Suspension aus Schritt (b) zur äußeren Düse der Doppeldüse;
e) gleichzeitiges Ausstoßen der Lösung, Emulsion oder Suspension aus Schritt (a) und derjenigen aus Schritt (b) aus der Doppeldüse unter Bedingungen, die eine wirksame Tropfenbildung ermöglichen; und
f) Unterwerfen der in Schritt (e) gebildeten Tropfen unter Bedingungen zum Bewirken der Kapselwandbildung.

Bevorzugt, insbesondere zur gleichzeitigen Lösung der oben genannten zweiten Aufgabe der besseren Bioverfügbarkeit, wird in Schritt (a) eine Lösung der zu verkapselnden Substanz(en) in einem nicht-wässrigen Lösungsmittel hergestellt.

Vorzugsweise ist bei der Erfindung vorgesehen, dass das in Schritt (b) verwendete Medium wässrig ist.

Weiter ist vorgesehen, dass die Viskosität der in Schritt (a) hergestellten Lösung, Emulsion oder Suspension angepaßt wird an diejenige der in Schritt (b) hergestellten.

Bevorzugt beträgt die Viskosität der in Schritt (a) hergestellten Lösung, Emulsion oder Suspension höchstens 200 mPa.s.

In einer besonderen Ausführungsform der Erfindung ist vorgesehen, dass die zu verkapselnde Substanz(en) wenigstens einen pharmazeutischen Wirkstoff umfasst, wie bspw. Praziquantel oder Epsiprantel.

Die in Schritt (b) hergestellte Lösung, Emulsion oder Suspension umfasst vorzugsweise ein wasserlösliches Salz der Alginsäure, besonders bevorzugt Natriumalginat.

In einer weiteren Ausführungsform der Erfindung ist vorgesehen, dass die gebildeten Mikrokapseln, ggf. nach Trocknung, einer Filmbeschichtung unterworfen werden.

Weiterhin betrifft die Erfindung eine mit dem erfindungsgemäßen Verfahren hergestellte Mikrokapsel, bestehend aus einer inneren Phase, die einen pharmazeutischen Wirkstoff, ausgewählt aus der Gruppe, die aus Praziquantel und Epsiprantel besteht, in Lösung, Emulsion oder Suspension enthält, und einer die innere Phase vollständig umhüllenden Kapselwand, die für den in der inneren Phase enthaltenen pharmazeutischen Wirkstoff undurchlässig ist.

Schließlich ist die Erfindung gerichtet auf eine pharmazeutische Zusammensetzung, die die erfindungsgemäßen Mikrokapseln in einer für orale Verabreichung geeigneten Zubereitungsform enthält.

Die Erfindung betrifft außerdem die Verwendung von erfindungsgemäßen Mikrokapseln, die in der inneren Phase Praziquantel bzw. Epsiprantel enthalten, zur Herstellung eines Arzneimittels zur Wurmbehandlung bei Haustieren, insbesondere Pferden, Hunden, oder Katzen.

Anhand der Geschmacks-Modellsubstanzen Praziquantel und Epsiprantel konnte gezeigt werden, dass das erfindungsgemäße Verfahren, das grundsätzlich bekannt ist und in der DE 197 52 585 A1 und WO 99/44735 zum Verkapseln von mikrobiellen, pflanzlichen und tierischen Zellen bzw. von biologischen und chemischen Substanzen beschrieben ist, überraschenderweise zur Geschmacksmaskierung von hinsichtlich ihres Geschmacks besonders kritischen pharmazeutischen Wirkstoffen verwendet werden kann, da mit diesem Verfahren eine zumindest nahezu 100%ige Effektivität der Geschmacksmaskierung erreicht werden kann.

Darüber hinaus erhöht die Einbettung des pharmazeutischen Wirkstoffes in der inneren Phase in einen hydrophilen Träger die Bioverfügbarkeit des Wirkstoffes bei oraler Verabreichung.

Am Beispiel der Modellsubstanz Praziquantel sollen im folgenden die besonderen Probleme dargestellt und die Möglichkeiten zu deren Lösung durch Anwendung des erfindungsgemäßen Verfahrens beschrieben werden, wobei analoge Überlegungen für andere problematische Wirkstoffe, wie bspw. Epsiprantel, gelten.

Die Geschmacksmaskierung von Praziquantel ist besonders schwierig aufgrund der besonderen Stoffeigenschaften dieser Substanz, nämlich starke Bitterkeit, schlechte Fließeigenschaften, schlechte Löslichkeit besonders im wässrigen Milieu und nadelförmige Kristallform. Die Verkapselung von Praziquantel ist auch problematisch aufgrund der notwendigen Eigenschaften der Kapselwand: einerseits sollen die Wände der Mikrokapseln im Mund des Patienten unlöslich sowie zur Herstellung einer Arzneiform widerstandsfähig sein, andererseits soll der Wirkstoff trotz seiner relativ schlechten intrinsischen Freisetzungsrate aufgrund seiner Indikation (Wurmmittel) im Magen/Darmbereich angemessen freigesetzt werden.

Die bekannten Verfahren zur Mikroverkapselung sind in praxi zur Verkapselung von Praziquantel aus verschiedenen Gründen nicht geeignet. Die Umhüllung ist, insbesondere wegen der besonderen Kristallform von Praziquantel, nie quantitativ, es kommt häufig zu Partikelagglomerationen und es bestehen meist erhebliche Schwierigkeiten bei Veränderungen der Chargengröße. Darüber hinaus finden sich auch in oberflächennahen Regionen der Kapselwand oder an der Außenseite der Kapselwand Spuren von Wirkstoff. Schließlich sind so hergestellte Kapseln nicht ideal rund und zeigen eine relativ breite und nicht optimal zu steuernde Partikelgrößenverteilung, was bei der Weiterverarbeitung zu Problemen führen kann.

Die geschilderten Probleme können durch Anwendung des erfindungsgemäßen Verfahrens vollständig gelöst werden. Dabei wird das Praziquantel in einem ersten Schritt in Lösung gebracht. Eine solche Maßnahme würde vom Fachmann zwar ins Auge gefasst werden, um die Bioverfügbarkeit von Praziquantel zu erhöhen, da dieses aus Lösung schneller und effektiver im Magen freigesetzt wird als aus der grobkristallinen Form, andererseits hätte der Fachmann aber diesen Weg deshalb nicht in Erwägung gezogen, weil der extrem bittere Geschmack von Praziquantel in gelöster Form noch deutlich verstärkt wird. Das Vorliegen des Wirkstoffes in Lösung stellt daher bei der Zielsetzung einer Geschmacksmaskierung einen ungewöhnlichen Weg dar, der für den Fachmann kontraindiziert wäre.

Obgleich Praziquantel in lipophilen Lösungsmitteln besser löslich ist als in hydrophilen, sollte versucht werden, Praziquantel in wässriger "Lösung" zur Verfügung zu stellen, insbesondere weil die Verfügbarkeit aus einer solchen üblicherweise besser ist als aus einer hydrophoben Lösung. Eine reine Suspension von Praziquantel ist für das vorliegende Verfahren in bezug auf eine homogene Wirkstoffverteilung und eine gute Kapselwandbildung aufgrund der nadeiförmigen Kristalle von Praziquantel problematisch.

Als besonders geeignete Methode zur Lösung von Praziquantel hat sich in den bisherigen Versuchen die Herstellung eines Coevaporates aus einer primären Öl-in-Wasser-Emulsion herausgestellt, wodurch eine energetisch günstige feste Praziquantel-Dispersion erhalten wird.

Bei der Herstellung der primären Öl-in-Wasser-Emulsion kann die lipophile Phase ein Öl oder Fett, aber auch ein Lösungsmittel wie Chloroform, Benzylalkohol oder Ethanol sein. Die erfindungsgemäße Ölphase besteht bevorzugt aus Dichlormethan, wobei der Wirkstoff in einer Konzentration von bis zu 70 Gew.-%, besonders bevorzugt 20 - 50 Gew.-%, inkorporiert wird.

Die hydrophile Phase besteht aus einem organischen Hydrogelbildner und Wasser. Als Gelbildner können hierfür bekannte Stoffe wie Gelatine, Carrageenan, Agar, Pektin, Gellan oder Methylcellulose eingesetzt werden. Die erfindungsgemäße hydrophile Phase besteht bevorzugt aus einer wässrigen Lösung von Natriumalginat in einer Konzentration von 1 bis 5 Gew.-%, besonders bevorzugt 2 Gew.-%.

Zur Verbesserung der Freisetzung des Wirkstoffes können der hydrophilen Phase Zerfallsmittel zugesetzt werden, wie z.B. Stärke oder Cellulose.

Zur Verbesserung der Löslichkeit des Wirkstoffes und/oder der Emulsions- oder Suspensions-Stabilität können außerdem Cosolventien oder/und Emulgatoren zugesetzt werden. Bevorzugt sind Polyethylengylkol (PEG) 400 (1 - 10 Gew.-%, besonders bevorzugt 5 Gew.-%) und der Emulgator Tween 80 (1 - 3 Gew.-%, besonders bevorzugt 1,5 Gew.-%).

In diesem Zusammenhang sei darauf hingewiesen, dass die innere Phase der erfindungsgemäß hergestellten Mikrokapsel auch eine übersättigte Lösung sein kann.

Beim erfindungsgemäßen Verfahren erfolgt schließlich der Einschluss des oben beschriebenen Mikrokapselkernes (Lösung (a)) durch Umhüllung mit Lösung (b).

Zur Ausbildung der Kapselwand wird im speziellen Beispiel eine wässrige Lösung von Natriumalginat hergestellt, welche stabilisiert werden kann z.B. mit Talkum oder Titandioxid, besonders bevorzugt mit Tricalciumphosphat.

Selbstverständlich können zur Herstellung der Kapselwand auch andere gel-/filmbildenden Lösungen oder Suspensionen verwendet werden wie bspw. Gelatine, Carrageenan, Gellan, Agar oder Mischungen mit Polyacrylharzen oder Cellulosen, um nur einige zu nennen.

Zur Härtung des Hüllmaterials werden die noch flüssigen Mikrotropfen in ein Härtungsbad eingebracht, im vorliegenden Fall eine Calciumchloridlösung. Durch Ionenaustausch kommt es zur Bildung und Vernetzung von Calciumalginat in der Außenschicht der Tropfen und somit zur Kapselwandbildung.

Selbstverständlich ist beim erfindungsgemäßen Verfahren das Aufbringen von Versiegelungsschichten aus z.B. Polylysin und/oder Chitosan möglich.

Die oben geschilderte Methode führt zu nassen Mikrokapseln. Diese werden schonend getrocknet, bevorzugt mittels Sprühtrocknung.

Zur Erhöhung der Stabilität gegenüber Speichelflüssigkeit bzw. zur Einstellung der gewünschten Wirkstoff-Freisetzung können die gehärteten Kapseln darüber hinaus noch mit den bekannten Methoden und Materialien befilmt werden. Eine häufig verwendete Methode ist z.B. das Wirbelschichtverfahren mit Filmbildnern wie Cellulosederivaten, Methacrylsäurecopolymeren, Polyvinylderivaten, Wachsen und natürlichen Gemischen. Nach Bedarf können wasserlösliche Filme mit unlöslichen kombiniert werden oder/und der Rezeptur übliche Hilfsstoffe wie Weichmacher und Emulgatoren zugesetzt werden.

Zur Befilmung der erfindungsgemäßen Kapseln wird bevorzugt die Hot-Melt-Coating-Technik angewandt, eine besonders einfache, umweltfreundliche und ökonomische Methode. Es hat sich außerdem überraschenderweise gezeigt, dass sich auch mit Wachsen eine gesteuerte Freisetzung erzielen läßt. Diese wird dadurch erreicht, dass im ersten Schritt eine hydrophilisierte Schmelze, z.B. eine Mischung aus Polyethylenglykol oder Stearoyl-Macrogolglycerid (Gelucire 50/13) einerseits und Glycerol-Dibehenat (Compritol 888) andererseits, aufgetragen wird und im zweiten Schritt eine Schmelze von wasserunlöslichem Glycerol-Distearat (Precirol Ato 5).

Das optimale Verhältnis von Geschmacksmaskierung einerseits und erwünschtem Freisetzungsverhalten des Wirkstoffes im Magen andererseits wurde erzielt, wenn die hydrophile Komponente und des Gycerol-Distearat im Verhältnis 10:90 bis 40:60, bevorzugt 20:80 aufgebracht wurden.

Nach Bedarf können der Rezeptur Zerfallsmittel wie Natriumhydrogencarbonat oder hochdisperses Siliziumdioxid bzw. oberflächenaktive Substanzen wie Tween zugesetzt werden.

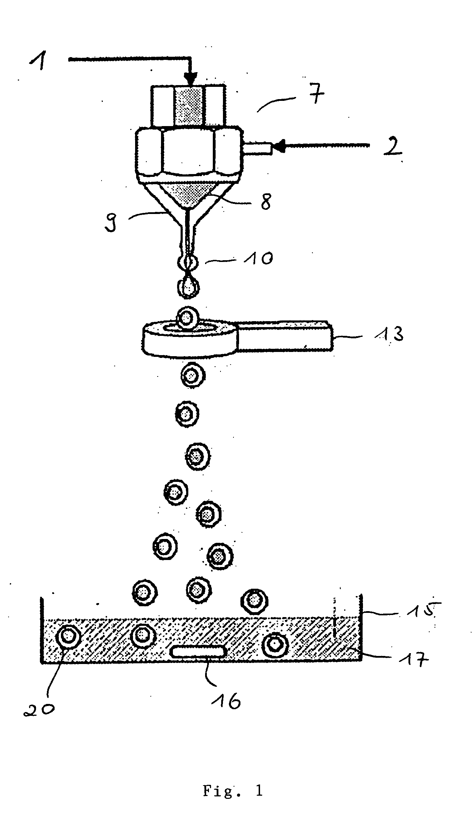

Das erfindungsgemäße Verfahren soll anhand der beigefügten Zeichnung näher erläutert werden. Eine Lösung, Emulsion oder Suspension 1 der zu verkapselnden Substanz (beispielsweise Praziquantel) und eine Lösung, Emulsion oder Suspension 2 des kapselwandbildenden Materials werden zu einer Doppeldüse 7 zugeführt, indem Lösung 1 in die innere Düse 8 und Lösung 2 (im vorliegenden Beispiel Natriumalginatlösung) in die äußere Düse 9 zugeführt wird.
Vorzugsweise ist die Doppeldüse mit einem Pulsator (nicht dargestellt) verbunden, um die Düse in Pulsation zu versetzen und dadurch eine effektive Tropfenbildung zu bewirken, wie näher beschrieben in der DE 197 52 585 A1, auf die insofern vollinhaltlich Bezug genommen wird.

Die Flüssigkeiten treten durch eine sehr präzise ausgebohrte konzentrische Düsenöffnung aus und zerfallen nach dem Austritt aus der Düse 7 in konzentrische, gleich große Tropfen 10, bei denen die innere Phase vollständig von der äußeren Phase umschlossen ist. Diese Tropfen können vorzugsweise durch ein elektrisches Feld zwischen der Düse 7 und der Elektrode 13 hindurchgehen, das den Tropfen eine Oberflächenladung verleiht. Die elektrostatischen Abstoßungskräfte verhindern ein Koagulieren der Tropfen im Flug und die Aggregationsbildung im Auffangbad. Ein derartiges Verfahren ist detaillierter beschrieben in der WO 99/44735, auf die insofern ebenfalls vollinhaltlich Bezug genommen wird.

Die Tropfengröße wird gesteuert durch verschiedene Parameter, wie Vibrationsfrequenz, Düsengröße, Durchflussgeschwindigkeit und physikalische Eigenschaften der Wandbildenden Materialien. In Abhängigkeit von den Variablen können 50 bis 4.000 Tropfen pro Sekunde erzeugt werden. Diese werden im vorliegenden Beispiel in einem Sammelbehälter 15 mit einem Rührkern 16 aufgefangen, der ein Härtungsbad 17 enthält, im vorliegenden Fall eine Calciumchloridlösung. Durch Ionenaustausch kommt es zur Bildung und Vernetzung von Calciumalginat in der Außenschicht der Tropfen und somit zu einer Wandbildung.

Die Mikrokapseln 20 mit zumindest semistabiler Kapselwand können aus dem Sammelbehälter 15 entfernt und ggf. noch gewaschen werden. Anschließend werden die Kapseln getrocknet und beschichtet.

Im Falle von Praziquantel, das an Hunde oder Katzen verabreicht werden soll, erfolgt in einem abschließenden Schritt die Herstellung eines Oleogels mit den wirkstoffhaltigen Mikrokapseln. Die Verabreichung dieses Oleogels an Hunde und insbesondere an Katzen führt zu einer völlig unproblematischen Aufnahme. In keinem Fall verweigerte eines der Testtiere die Aufnahme der erforderlichen Menge.

Die Mikroverkapselung von Praziquantel oder Espiprantel_mit dem erfindungsgemäßen Verfahren stellt somit erstmalig eine zu 100 Gew.-% geschmacksmaskierte, pharmazeutische Zubereitung dieser Wirkstoffe zur Verfügung, die sich darüber hinaus auch noch durch verbesserte Bioverfügbarkeit auszeichnet durch (Auflösung der kristallform des Wirkstoffes).

Die in der vorstehenden Beschreibung, in den Ansprüchen sowie in den Zeichnungen offenbarten Merkmale der Erfindung können sowohl einzeln als auch in beliebiger Kombination für die Verwirklichung der Erfindung in ihren verschiedenen Ausführungsformen wesentlich sein.

## Patentansprüche

1. Verfahren zur Geschmacksmaskierung von Substanzen durch Mikroverkapselung mit den folgenden Schritten:
a) Herstellung einer Lösung, Emulsion oder Suspension der Wirkstoff(e) in einem geeigneten Medium;
b) Herstellung einer Lösung, Emulsion oder Suspension, die wenigstens ein kapselwandbildendes Material enthält, in einem geeigneten Medium;
c) Zuführen der Lösung, Emulsion oder Suspension aus Schritt (a) zur inneren Düse einer Doppeldüse;
d) Zuführen der Lösung, Emulsion oder Suspension aus Schritt (b) zur äußeren Düse der Doppeldüse; und
e) gleichzeitiges Ausstoßen der Lösung, Emulsion oder Suspension aus Schritt (a) und derjenigen aus Schritt (b) aus der Doppeldüse unter Bedingungen, die eine wirksame Tropfenbildung ermöglichen; und
f) Unterwerfen der in Schritt (e) gebildeten Tropfen unter Bedingungen zum Bewirken der Kapselwandbildung.

2. Verfahren nach Anspruch 1, **dadurch gekennzeichnet, dass** in Schritt (a) eine Lösung der zu verkapselnden Substanz(en) in einem nicht-wässrigen Lösungsmittel hergestellt wird.

3. Verfahren nach einem Anspruch 1 oder 2, **dadurch gekennzeichnet, dass** das in Schritt (b) verwendete Medium wässrig ist.

4. Verfahren nach einem der vorangehenden Ansprüche, **dadurch gekennzeichnet, dass** die Viskosität der in Schritt (a) hergestellten Lösung, Emulsion oder Suspension angepaßt wird an diejenige der in Schritt (b) hergestellten.

5. Verfahren nach einem der vorangehenden Ansprüche, **dadurch gekennzeichnet, dass** die Viskosität der in Schritt (a) hergestellten Lösung, Emulsion oder Suspension höchstens 200 mPa.s beträgt.

6. Verfahren nach einem der vorangehenden Ansprüche, **dadurch gekennzeichnet, dass** die zu verkapselnde Substanz(en) wenigstens einen pharmazeutischen Wirkstoff umfasst.

7. Verfahren nach Anspruch 6, **dadurch gekennzeichnet, dass** der pharmazeutische Wirkstoff Praziquantel ist.

8. Verfahren nach Anspruch 6, **dadurch gekennzeichnet, dass** der pharmazeutische Wirkstoff Epsiprantel ist.

9. Verfahren nach einem der vorangehenden Ansprüche, **dadurch gekennzeichnet, dass** die in Schritt (b) hergestellte Lösung, Emulsion oder Suspension ein wasserlösliches Salz der Alginsäure umfasst.

10. Verfahren nach Anspruch 9, **dadurch gekennzeichnet, dass** das wasserlösliche Salz der Alginsäure Natriumalginat ist.

11. Verfahren nach eine der vorangehenden Ansprüche, **dadurch gekennzeichnet, daß** die in Schritt (f) gebildeten Mikrokapseln, ggf. nach Trocknung, einer Filmbeschichtung unterworfen werden.

12. Mikrokapsel, herstellbar mit einem Verfahren nach einem der vorangehenden Ansprüche, bestehend aus einer inneren Phase, die einen pharmazeutischen Wirkstoff, ausgewählt aus der Gruppe, die aus Praziquantel und Epsiprantel besteht, in Lösung, Emulsion oder Suspension enthält, und einer die innere Phase vollständig umhüllenden Kapselwand, die für den in der inneren Phase enthaltenen pharmazeutischen Wirkstoff undurchlässig ist.

13. Pharmazeutische Zusammensetzung, die Mikrokapseln nach Anspruch 12 in einer für orale Verabreichung geeigneten Zubereitungsform enthält.

14. Verwendung von Mikrokapseln nach Anspruch 12, die in der inneren Phase Praziquantel enthalten, zur Herstellung eines Arzneimittels zur Wurmbehandlung bei Haustieren, insbesondere Pferden, Hunden oder Katzen.

15. Verwendung von Mikrokapseln nach Anspruch 12, die in der inneren Phase Epsiprantel enthalten, zur Herstellung eines Arzneimittels zur Wurmbehandlung bei Haustieren, insbesondere Pferden, Hunden oder Katzen.
